# EUROPEAN PATENT SPECIFICATION

(11) **EP 1 954 107 B1**
(45) Date of publication and mention of the grant of the patent: **05.05.2010**
(21) Application number: 08100872.4
(22) Date of filing: 24.01.2008
(51) Int. Cl.: H05B 41/292

(54) **Ignition limited illuminator**
Beleuchtungsvorrichtung mit begrenzter Zündung
Illuminateur à allumage limité

(30) Priority: 26.01.2007 US 886752 P
(43) Date of publication of application: 06.08.2008
(73) Proprietor: Alcon, Inc., 6331 Hünenberg (CH)
(72) Inventor: Papac, Michael, Tustin, CA 92780 (US); Mercado, Fred, Laguna Hills, CA 92653-5615 (US)
(74) Representative: Hanna, Peter William Derek

(56) References cited:
- US-A1- 2002 113 560
- US-A1- 2003 203 699
- US-B1- 6 307 332

## Description

### TECHNICAL FIELD OF THE INVENTION

The present invention relates generally to the field of illumination systems. In particular, the present invention relates to ophthalmic illumination systems and, more particularly, to a method and system for enhancing the useful lifetime of an ophthalmic illumination system.

### BACKGROUND OF THE INVENTION

Many ophthalmic surgical procedures require illuminating a portion of a patient's eye so that a surgeon can observe the surgical site. Various different types of instruments are known and available for use by an ophthalmic surgeon to illuminate the interior of the eye. Ophthalmic illuminators are commonly used for this purpose.

The handheld (probe) portion of a typical ophthalmic illuminator comprises a handle having a projecting tip and a length of optical fiber that enters a proximal end of the handle and passes through the handle and the tip to a distal end of the tip, from which light traveling along the optical fiber can project. The proximal end of the optical fiber can be optically coupled to a light source, such as in a high brightness illuminator, to receive the light that is transmitted through the fiber. These types of illuminator probes are typically used by inserting the probe tip through a small incision in the eye. In this way, light from the illuminator light source is carried along the optical fiber, through the handpiece and emitted from the distal end of the probe (fiber) to illuminate the surgical site for the surgeon. Ophthalmic illumination systems that use a length of optical fiber to carry and direct light from a light source to a surgical site are well known in the art.

Such an ophthalmic illumination system typically comprises the handheld probe, to deliver illumination from a light source housed in an enclosure, the enclosure typically housing the light source and associated optics that guide light from the light source to the optical fiber of the probe, a power supply, electronics with signal processing, and associated connectors, displays and other interfaces as known to those having skill in the art. While some ophthalmic illumination systems use other types of lamps as a light source, a preferred light source is an arc lamp, such as a xenon arc lamp.

Ophthalmic illumination system xenon arc lamps are typically designed to operate for a fixed amount of time (lamp lifetime). The lamp lifetime limit is designed to correspond to a point at which the lumen output of the lamp is expected to decrease below a desired output specification, and which may also correspond to a lamp bulb age beyond which continued operation of the lamp is much more likely to result in catastrophic failure of the arc lamp. However, in certain types of illuminator systems, such as the Alcon High Brightness Illuminator ("AHBI") manufactured by Alcon Laboratories, Inc. of Fort Worth, Texas, the illuminator light source is designed to provide a higher lumen output for a much longer duration of time than typical arc lamp illuminators. In such an illuminator system, the specified lamp lifetime limit can be reached before the lumen output degrades below the set specification, allowing the illuminator to perform adequately throughout the lifetime of the lamp. Decreased light output is thus no longer available as an indicator that a lamp bulb has aged beyond a point where continued operation is more likely to result in catastrophic failure.

In an illuminator system such as the AHBI, once a lamp bulb reaches a certain age (e.g., 400 hours), a software mechanism alerts a user that the bulb should be changed. To prevent the loss of illumination during a surgical procedure, this software mechanism can be overridden. A further software lock may also be used to prevent ignition of the lamp bulb, but this too may be overridden. Subsequent use of the illuminator can cause the lamp bulb to operate in a dangerous regime where the potential for catastrophic failure increases. A catastrophic failure of the bulb while installed in the illuminator has potential to destroy the illuminator and potentially other modules within an ophthalmic surgical system housing the illuminator. No hardware lock exists in such illuminators to physically prevent ignition of illuminator bulbs that have aged beyond a designated safe lifetime.

US-2002/0113560 A1 (Edwards et al) describes a lighting apparatus having a means for counting and recording an actual amount of time that the light source has been used and/or a temperature of the light source. The power supply may be shut off if the time exceeds a predetermined time, or if the temperature exceeds a predetermined temperature.

Therefore, a need exists for a method and system for hardware limiting the ignition of an arc lamp, such as a xenon arc lamp, of an ophthalmic illumination system that can reduce or eliminate the problems of prior art ophthalmic illumination systems discussed above.

### BRIEF SUMMARY OF THE INVENTION

The present invention provides a method and system for hardware limiting the ignition of an arc lamp in accordance with the claims which follow. One embodiment of the present invention is an ophthalmic illuminator, comprising: an illumination source having a desired useful lifetime; and an ignition system operable to provide an ignition pulse to ignite the illumination source, wherein the ignition pulse voltage is limited to prevent ignition of the illumination source beyond the desired useful lifetime. The desired useful lifetime of the illumination source can be an arbitrarily selected lifetime that is less than an expected illumination source lifetime based on manufacturer's specifications for the illumination source. The ignition pulse voltage limit can be a threshold voltage, wherein the threshold voltage is a minimum voltage required to ignite the illumination source near the end of the desired useful lifetime. The illuminator can further comprise an alerting system operable to alert a user to replace the illumination source if the ignition pulse fails to ignite the illumination source.

Other embodiments of this present invention can include a method for hardware limiting the ignition of an arc lamp of an ophthalmic illuminator by imposing a limit on the energy deposited on the lamp electrodes during an ignition pulse in accordance with the teachings of this invention. One embodiment of a method of operating an ophthalmic illuminator in accordance with the present invention comprises: providing an ophthalmic illuminator illumination source; determining a desired useful lifetime of the illumination source; and providing an ignition system operable to provide an ignition pulse to ignite the illumination source, wherein the ignition pulse voltage is limited to prevent ignition of the illumination source beyond the desired useful lifetime. The desired useful lifetime of the illumination source can be determined by arbitrarily selecting a desired lifetime less than an expected illumination source lifetime based on manufacturer's specifications for the illumination source. The method can further comprise, if the ignition pulse fails to ignite the illumination source, alerting a user to replace the illumination source.

Embodiments of this invention can be implemented within a surgical machine or system for use in ophthalmic or other surgery. In particular, it is contemplated that the method and system for hardware limiting the ignition of an arc lamp of an ophthalmic illumination system of this invention can be implemented in, or incorporated into, any ophthalmic illumination system in which it is desirable to efficiently and safely couple an arc lamp light source, such as a mercury-xenon, mercury vapor or metal halide light source to an optical beam delivery system, such as a small diameter optical fiber. Other uses for the method and system of this invention will be apparent to those having skill in the art.

### BRIEF DESCRIPTION OF THE SEVERAL VIEWS OF THE DRAWINGS

A more complete understanding of the present invention and the advantages thereof may be acquired by referring to the following description, taken in conjunction with the accompanying drawings, in which like reference numbers indicate like features and wherein:
FIGURE 1 is a diagrammatic representation of one embodiment of an enhanced high brightness ophthalmic illuminator system of the present invention;
FIGURE 2 is a more detailed diagrammatic representation of a portion of illuminator system 10 of FIGURE 1;
FIGURE 3 is a diagrammatic representation of a lamp ignition system for an illumination source in accordance with the present invention;
FIGURES 4A and 4B are images showing the electrode gap of an exemplary Osram XBO 75 watt short arc bulb illumination source at 0 hours and 1200 hours;
FIGURE 5 is a graph showing the ignition voltage required to ignite an exemplary arc lamp bulb as the arc lamp bulb ages;
FIGURE 6 is a graph showing a hypothetical waveform trace to illustrate the expected ignition pulses required to ignite a new lamp (left trace) and an old lamp (right trace) and an exemplary hardware limited ignition threshold voltage; and
FIGURE 7 is a block diagram illustrating the steps of one embodiment of the method of the present invention.

### DETAILED DESCRIPTION OF THE INVENTION

Preferred embodiments of the present invention are illustrated in the FIGUREs, like numerals being used to refer to like and corresponding parts of the various drawings.

The various embodiments of the method and system of the present invention provide for hardware limiting the ignition of an arc lamp illumination source of an ophthalmic illumination system to prevent catastrophic failure of an aged arc lamp illumination source and to indicate the presence of a failed bulb. An arc lamp is ignited by "striking" (applying) a high voltage pulse at the arc lamp bulb electrodes, initiating a discharge between the two electrodes which will stabilize into an arc that emits visible light for illumination. The energy required to ignite an arc lamp with a given internal gas pressure and composition is directly proportional to the spacing between the lamp electrodes (arc gap). For a given lamp, the electrode spacing is directly proportional to the age of the lamp due to erosion of the electrodes (primarily at the cathode) over time during operation. By imposing a limit on the energy applied at the arc lamp electrodes during the ignition pulse, the embodiments of the method and system of this invention can provide an upper limit to the spacing (erosion) of the lamp electrodes and hence on the lamp operating lifetime.

The embodiments of the method and system of the present invention can provide for hardware limiting the ignition pulse provided to ignite an arc lamp. The ignition pulse provided can be tailored to ensure that arc lamp bulbs that have been operated beyond a desired threshold lifetime will not ignite (due to too large electrode spacing), but lamps younger than the lifetime threshold will ignite. This can be accomplished in the electronics of an igniter and/or power supply module or ballast by, for example, imposing an upper limit to the ignition pulse voltage or by imposing a limit to the energy supplied by the igniter during the ignition pulse. Either of these limits can be set such that lamps with a large enough arc gap (i.e., lamps that have been operated beyond a desired lifetime) can no longer be ignited, thereby providing an upper limit to the spacing between the arc lamp electrodes and, hence, to the lamp operating lifetime. In this way, the embodiments of the method and system of the present invention can provide a hardware safety mechanism to reduce or prevent the instances of catastrophic failure of an aged arc lamp and the attending damage to the illuminator or surgical system. Such a hardware mechanism is designed so that it cannot be overridden by system software.

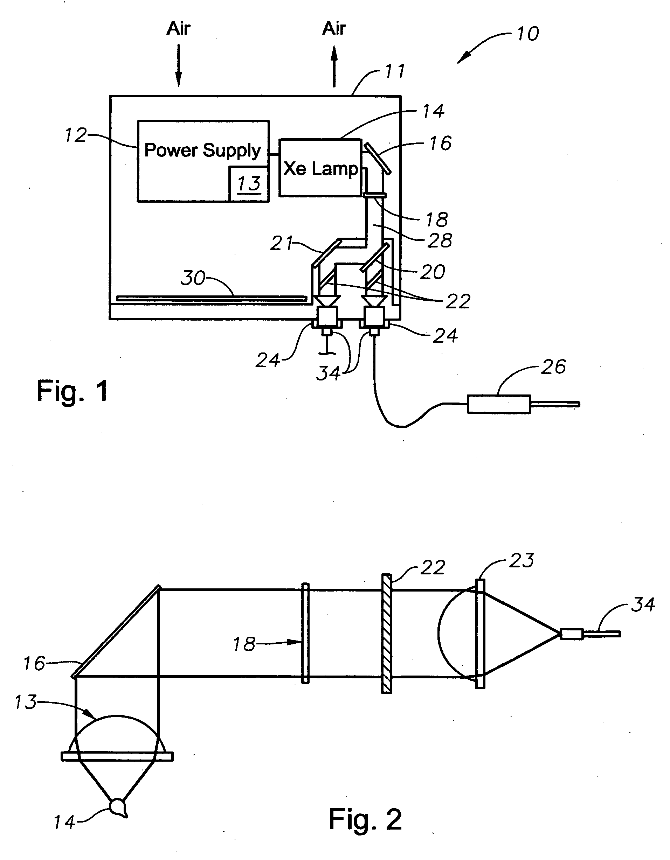

FIGURE 1 is a diagrammatic representation of a high brightness ophthalmic illuminator system in accordance with the present invention. Illuminator system 10 comprises power supply 12, which can include an illuminator ignition system 300, illumination source 14, cold mirror 16, a hot mirror 18, a beam splitter 20, mirror 21, optical fiber ports 24 and attenuators 22. Illuminator system 10 also can comprise one or more optical fiber probes 26 for receiving and transmitting light from illumination source 14 to a surgical site. Optical fiber probes 26 comprise the handheld portion of the illuminator system 10, including optical fiber 34, which is optically coupled to the illumination source 14 within enclosure 11. High brightness illuminator system 10 is exemplary only and is not intended to limit the scope of the present invention in any way. The embodiments of the present invention can be used to hardware limit the ignition of an arc lamp illumination source 14 of any such ophthalmic illuminator, medical laser, or any other system or machine in which it is desirable to hardware limit the ignition of an arc lamp illumination source.

Illumination source 14 of illuminator system 10 in this example comprises a xenon lamp, but it can comprise any suitable light source as known to those having skill in the art in which the cathode degrades with age affecting the arc spacing. Xenon lamp 14 emits light beam 28, which is directed along the optical path comprising cold mirror 16, hot mirror 18, beam splitter 20, mirror 21, attenuators 22, and optical fiber ports 24. In this example, beam splitter 20 splits light beam 28 into two optical paths to provide for two optical probes 26 if desired. Cold mirror 16 and hot mirror 18 combine to remove the infrared and UV components of light beam 28 (heat) and provide a cool visible light beam 28 to the downstream optical components, as will be familiar to those skilled in the art. Attenuators 22 attenuate optical beam 28. Attenuators 22 can each be custom designed for its respective optical path and need not be identical, though they can be. Further, each attenuator 22 can be independently controlled via, for example, PCB 30.

Although high brightness illuminator system is shown comprising two optical fiber ports 24 (with aspheric lenses or other focusing elements) , it will be obvious to those having skill in the art that a single optical port 24 or multiple optical ports 24 can be implemented within illuminator system 10. Illuminator system 10 further comprises a printed circuit board ("PCB") 30, or its electronic equivalent, to provide signal processing and control functions. PCB 30 can be implemented in any manner and configuration capable of performing the desired processing and control functions described herein, as will be apparent to those having skill in the art. Optical ports 24 comprise a receptacle to receive the proximal end of an optical fiber 34 corresponding to a fiber probe 26, which is inserted into the high brightness illuminator enclosure 11 and optically coupled to illumination source 14 to direct light onto a desired site.

FIGURE 2 is a more detailed diagrammatic representation of a portion of illuminator system 10 of FIGURE 1. Light emitted from the illumination source 14 arc region (e.g., a xenon arc lamp) is collimated by the collimating lens 13, and filtered by the cold mirror 16, hot mirror 18 and attenuator 22. The light is then focused by the condensing lens 23 (which can be part of an optical fiber port 24) into optical fiber 34.

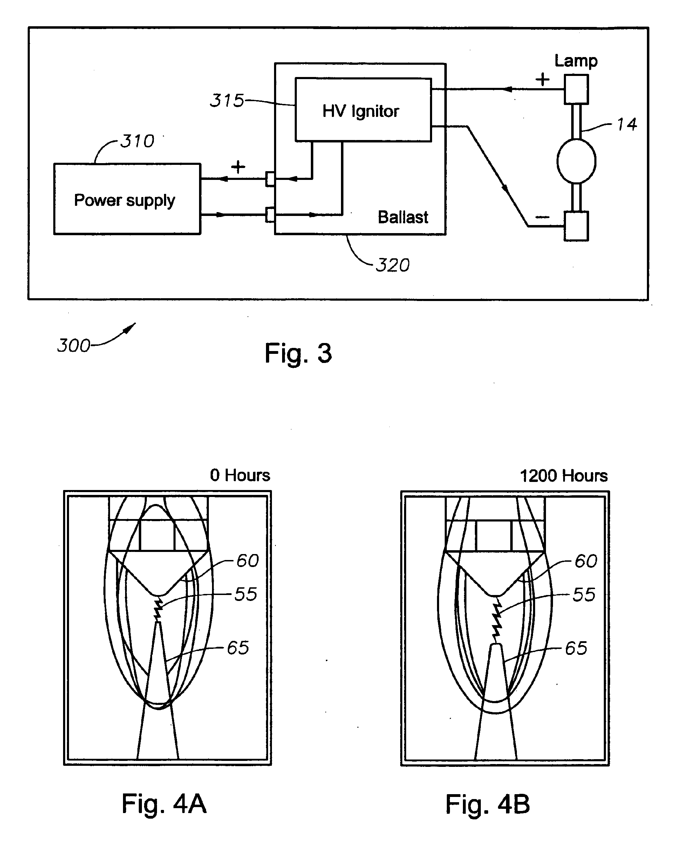

FIGURE 3 is a more detailed diagrammatic representation of lamp ignition system 300 for illumination source 14 in accordance with the present invention. Illumination source 14 in this example is an Osram 75W xenon bulb, but can be any suitable arc lamp bulb as will be known to those having skill in the art. Power supply 310 provides energy to HV igniter 315 of ballast 320. Power supply 310 can be separate from power supply 12 or integral to power supply 12. HV igniter 315 provides the ignition pulse to illumination source 14. Ignition system 300 can be designed such that the power supply 310 and/or HV igniter 315 (ballast 320) limit the energy or voltage supplied to the electrodes of illumination source 14 to a threshold voltage value in the manner described herein. The threshold voltage can be a minimum voltage required to ignite the illumination source near the end of the desired useful lifetime of illumination source 14. The desired useful lifetime of the illumination source can be determined by arbitrarily selecting a desired lifetime less than an expected illumination source lifetime based on manufacturer's specifications for the illumination source.

FIGUREs 4A and 4B are images showing the electrode gap of an exemplary Osram XBO 75 watt short arc bulb illumination source at 0 hours (FIG. 4A) and 1200 hours (FIG. 4B). The difference between the arc spacings in the two images illustrate the cathode erosion that occurs during operation from, in this example, zero to 1200 hours. As the illumination source 14 bulb ages, the tip of the cathode 65 erodes away, causing the tip of cathode 65 to move in a downward direction (for a typical installation) away from the anode 60 and to become blunter. As the cathode 65 erodes, the arc 55 grows in size, decreases in peak luminance, and also moves in the same direction as the cathode 65 away from anode 60, causing a monotonic and rapid decrease in the illuminator system light throughput. Although the examples provided herein involve an Osram 75W xenon bulb, the analysis and results are expected to be comparable for other such illumination sources.

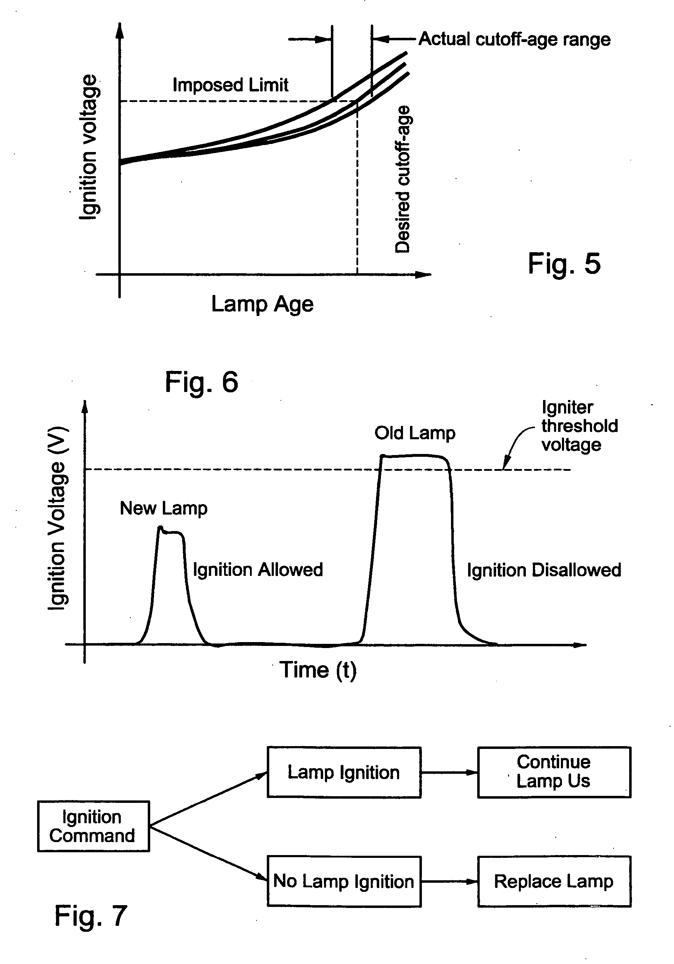

FIGURE 5 is a graph showing the ignition voltage required to ignite an exemplary arc lamp illumination source 14 as the arc lamp bulb ages. As can be seen from FIGURE 5, a desired threshold ignition voltage for a desired illumination source 14 lifetime can be determined from such a curve and designed into an ignition system 300, such as that of FIGURE 3.

FIGURE 6 is a graph showing hypothetical waveform traces to illustrate the expected ignition pulses required to ignite a new lamp (left trace) and an old lamp (right trace). FIGURE 6 also shows an exemplary hardware limited ignition threshold voltage. Imposing a threshold voltage limit on the ignition system 300 will make it operable to prevent the ignition of an illumination source 14 once the illumination source 14 electrode spacing increases to the point where the ignition voltage required to ignite the illumination source 14 exceeds the imposed threshold voltage (i.e., the desired illumination source 14 lifetime is exceeded).

FIGURE 7 is a block diagram illustrating some process steps of one embodiment of the method of the present invention. In particular, FIGURE 7 illustrates the underlying logic for an alerting system that can be a part of the embodiments of the present invention for alerting a user to replace illumination source 14 once its useful lifetime has been exceeded.

Various embodiments of the present invention thus provide for hardware limited ignition of an arc lamp illumination source of, for example, an ophthalmic illumination system. Further, the embodiments of this invention provide the advantage that they allow hardware to limit the operating lifetime of an arc lamp illumination source, thereby avoiding the catastrophic failure of the arc lamp during operation. The embodiments of the present invention can be incorporated into any arc lamp based illumination device, such as an ophthalmic illuminator.

## Claims

1. A method of operating an ophthalmic illuminator (10), comprising the steps of:
providing an ophthalmic illuminator illumination source (14);
determining a desired useful lifetime of the illumination source; and
providing an ignition system (300) operable to provide an ignition pulse to ignite the illumination source,
**characterized in that** the ignition pulse voltage is limited to a threshold voltage,
wherein the threshold voltage is selected as a minimum voltage required to ignite the illumination source (14) near the end of the desired useful lifetime.

2. The method of claim 1, wherein the illumination source (14) is a xenon arc lamp.

3. The method of claim 1, wherein determining the desired useful lifetime of the illumination source (14) comprises arbitrarily selecting a desired lifetime less than an expected illumination source lifetime based on manufacturer's specifications for the illumination source.

4. The method of claim 1, wherein the ignition system (300) comprises a power supply (12,310) and a high voltage igniter (315) for providing the ignition pulse.

5. The method of claim 1, further comprising the step of, if the ignition pulse fails to ignite the illumination source, alerting a user to replace the illumination source.

6. An ophthalmic illuminator (10), comprising:
an illumination source (14) having a desired useful lifetime; and
an ignition system (300) operable to provide an ignition pulse to ignite the illumination source,
**characterized in that** the ignition pulse voltage is limited to a threshold voltage, wherein the threshold voltage is selected as a minimum voltage required to ignite the illumination source (14) near the end of the desired useful lifetime.

7. The illuminator of claim 6, wherein the illumination source (14) is a xenon arc lamp.

8. The illuminator of claim 6, wherein the desired useful lifetime of the illumination source (14) is an arbitrarily selected lifetime that is less than an expected illumination source lifetime based on manufacturer's specifications for the illumination source.

9. The illuminator claim 6, wherein the ignition system (300) comprises a power supply (12,310) and a high voltage igniter (315) for providing the ignition pulse.

10. The illuminator of claim 6, further comprising an alerting system operable to alert a user to replace the illumination source (14) if the ignition pulse fails to ignite the illumination source.

## Patentansprüche

1. Verfahren zum Betreiben einer ophthalmischen Beleuchtungseinrichtung (10), das die Schritte aufweist:
Bereitstellen einer Beleuchtungsquelle (14) der ophthalmischen Beleuchtungseinrichtung;
Bestimmen der gewünschten nutzbaren Lebensdauer der Beleuchtungsquelle; und
Bereitstellen eines Zündsystems (300), das im Betrieb einen Zündimpuls zum Zünden der Beleuchtungsquelle bereitstellen kann,
**dadurch gekennzeichnet, dass** die Spannung des Zündimpulses auf eine Schwellenspannung begrenzt ist, wobei die Schwellenspannung als eine Mindestspannung gewählt wird, die zum Zünden der Beleuchtungsquelle (14) gegen Ende der gewünschten nutzbaren Lebensdauer erforderlich ist.

2. Verfahren nach Anspruch 1, bei dem die Beleuchtungsquelle (14) eine Xenon-Lichtbogenlampe ist.

3. Verfahren nach Anspruch 1, bei dem die Bestimmung der gewünschten nutzbaren Lebensdauer der Beleuchtungsquelle (14) die Wahl einer beliebigen gewünschten Lebensdauer aufweist, die kürzer ist als die erwartete Lebensdauer der Beleuchtungsquelle auf Basis der technischen Daten des Herstellers für die Beleuchtungsquelle.

4. Verfahren nach Anspruch 1, bei dem das Zündsystem (300) eine Spannungsversorgung (12, 310) und einen Hochspannungs-Zünder (315) zur Bereitstellung des Zündimpulses aufweist.

5. Verfahren nach Anspruch 1, das ferner den Schritt aufweist, in dem dann, wenn der Zündimpuls die Beleuchtungsquelle nicht zu zünden vermag, ein Benutzer darauf aufmerksam gemacht wird, die Beleuchtungsquelle zu ersetzen.

6. Ophthalmische Beleuchtungseinrichtung (10), mit:
einer Beleuchtungsquelle (14) mit einer gewünschten nutzbaren Lebensdauer; und
einem Zündsystem (300), das im Betrieb einen Zündimpuls zum Zünden der Beleuchtungsquelle bereitstellen kann,
**dadurch gekennzeichnet, dass** die Spannung des Zündimpulses auf eine Schwellenspannung begrenzt ist, wobei die Schwellenspannung als eine Mindestspannung gewählt wird, die zum Zünden der Beleuchtungsquelle (14) gegen Ende der gewünschten nutzbaren Lebensdauer erforderlich ist.

7. Beleuchtungseinrichtung nach Anspruch 6, bei der die Beleuchtungsquelle (14) eine Xenon-Lichtbogenlampe ist.

8. Beleuchtungseinrichtung nach Anspruch 6, bei der die gewünschte nutzbare Lebensdauer der Beleuchtungsquelle (14) eine beliebig gewählte Lebensdauer ist, die kürzer ist als die erwartete Lebensdauer der Beleuchtungsquelle auf Basis der technischen Daten des Herstellers für die Beleuchtungsquelle.

9. Beleuchtungseinrichtung nach Anspruch 6, bei der das Zündsystem (300) eine Spannungsversorgung (12, 310) und einen Hochspannungs-Zünder (315) zur Bereitstellung des Zündimpulses aufweist.

10. Beleuchtungseinrichtung nach Anspruch 6, ferner ein Alarmsystem aufweisend, das im Betrieb einen Benutzer darauf aufmerksam macht, die Beleuchtungsquelle (14) zu ersetzen, wenn der Zündimpuls die Beleuchtungsquelle nicht zu zünden vermag.

## Revendications

1. Procédé pour le fonctionnement d'un illuminateur ophtalmique (10), comprenant les étapes consistant à :
fournir une source d'éclairage d'illuminateur ophtalmique (14) ;
déterminer une durée de vie utile souhaitée de la source d'éclairage ; et
fournir un système d'allumage (300) fonctionnel pour fournir une tension d'allumage pour allumer la source d'éclairage,
**caractérisé en ce que** la tension d'impulsion d'allumage est limitée à une tension de seuil, la tension de seuil étant sélectionnée en tant que tension minimum requise pour allumer la source d'éclairage (14) peu avant la fin de la durée de vie utile souhaitée.

2. Procédé selon la revendication 1, dans lequel la source d'éclairage (14) est une lampe à arc au xénon.

3. Procédé selon la revendication 1, dans lequel la détermination de la durée de vie utile souhaitée de la source d'éclairage (14) comprend une sélection arbitraire d'une durée de vie souhaitée inférieure à une durée de vie présumée de la source d'éclairage, basée sur les spécifications du fabricant en ce qui concerne la source d'éclairage.

4. Procédé selon la revendication 1, dans lequel le système d'allumage (300) comprend une alimentation électrique (12, 310) et un allumeur haute tension (75) pour fournir l'impulsion d'allumage.

5. Procédé selon la revendication 1, comprenant en outre l'étape consistant à, si l'impulsion d'allumage échoue à allumer la source d'éclairage, avertir un utilisateur afin qu'il remplace la source d'éclairage.

6. Illuminateur ophtalmique (10), comprenant :
une source d'éclairage (14) ayant une durée de vie utile souhaitée ; et
un système d'allumage (300) fonctionnel pour fournir une impulsion d'allumage pour allumer la source d'éclairage,
**caractérisé en ce que** la tension d'impulsion d'allumage est limitée à une tension de seuil, la tension de seuil étant sélectionnée en tant que tension minimum requise pour allumer la source d'éclairage (14) peu avant la fin de la durée de vie utile souhaitée.

7. Illuminateur selon la revendication 6, dans lequel la source d'éclairage (14) est une lampe à arc au xénon.

8. Illuminateur selon la revendication 6, dans lequel la durée de vie utile souhaitée de la source d'éclairage (14) est une durée de vie sélectionnée de manière arbitraire qui est inférieure à une durée de vie présumée de la source d'éclairage, basée sur les spécifications du fabricant en ce qui concerne la source d'éclairage.

9. Illuminateur selon la revendication 6, dans lequel le système d'allumage (300) comprend une alimentation électrique (12, 310) et un allumeur haute tension (315) pour fournir l'impulsion d'allumage.

10. Illuminateur selon la revendication 6, comprenant en outre un système d'avertissement fonctionnel pour avertir un utilisateur afin qu'il remplace la source d'éclairage (14) si l'impulsion d'allumage échoue à allumer la source d'éclairage.
